Europäisches Patentamt

(19) European Patent Office

Office européen des brevets

(11) EP 0 631 756 B1

## (12) EUROPEAN PATENT SPECIFICATION

(45) Date of publication and mention
of the grant of the patent:
26.03.1997 Bulletin 1997/13

(51) Int. Cl.$^6$: **A61B 5/00**

(21) Application number: 93305036.1

(22) Date of filing: 28.06.1993

(54) **Monitoring apparatus**

Überwachungsgerät

Appareil de surveillance

(84) Designated Contracting States:
**DE GB**

(43) Date of publication of application:
**04.01.1995 Bulletin 1995/01**

(73) Proprietor: **HAMAMATSU PHOTONICS K.K.**
**Shizuoka-ken (JP)**

(72) Inventors:
• **Cope, Mark,**
c/o Univ.College London
London WC1E 6JA (GB)

• **Delpy, David Thomas,**
c/o Univ.College London
London WC1E 6JA (GB)

(74) Representative: **Rackham, Stephen Neil**
**GILL JENNINGS & EVERY,**
**Broadgate House,**
**7 Eldon Street**
**London EC2M 7LH (GB)**

(56) References cited:
EP-A- 0 501 283          EP-A- 0 527 703
WO-A-92/21280          US-A- 4 281 645
US-A- 4 380 240          US-A- 5 203 329

## Description

The present invention relates to an apparatus for non-invasively monitoring a selected activity in an object, and more particularly to an apparatus for non-invasively monitoring metabolism, blood flow, blood volume, hemoglobin content of blood, and the like of an in vivo organ such as the brain.

Many methods of irradiating an object with certain wavelength light and measuring the amount of light reflected or absorbed by the object to accurately analyze characteristics of the object are well known in the art. For example, oximeters have been used to measure the oxygenated fraction of hemoglobin in blood circulating in the body. A thin body part, such as an earlobe, a finger tip, or nose tip, is irradiated and the absorption of light after transmitting through or reflecting from the blood is measured. Recent advances in technology have produced devices such as described in U.S. Patent No. 4,281,645 for measuring changes in blood volume, the oxygenation state of hemoglobin, and the rate of blood flow in the brain, heart, kidney, other organs, in limbs, or other parts of a human or animal body characteristics by measuring the amount of light these components absorb. Such devices allow non-invasive, continuous, bedside monitoring of blood volume in tissue under investigation and blood oxygen levels, and for intermittent monitoring of blood flow rate, particularly in the head, as determined by relative levels of oxidized hemoglobin, $HbO_2$ and reduced hemoglobin, Hb.

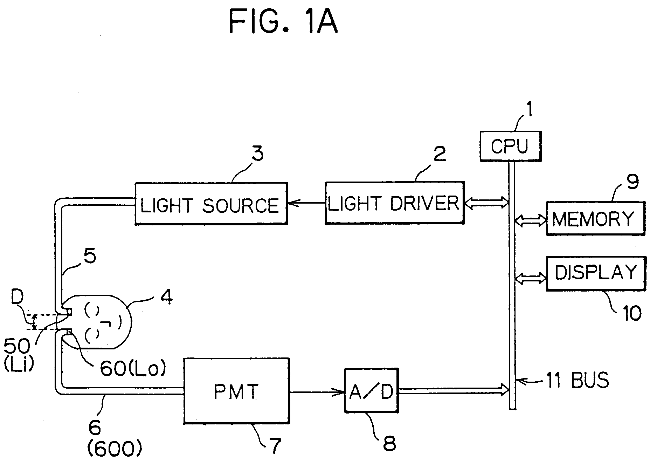

A conventional system for measuring amount of disoxygenated hemoglobin in the blood flowing in the brain as a function of absorption of 760 nm wavelength light is shown in Fig. 1A. The system includes a light irradiating optical-fiber bundle 5 and a light collecting optical-fiber bundle 6. One end of the light irradiating optical-fiber bundle 5 is optically connected to a light source 3. The other end surface of the light irradiating optical-fiber bundle 5 (which will be referred to as a "light irradiating surface 50," hereinafter) is affixed to the surface of the head 4 so as to define a light irradiating area $L_i$ of the head. One end of the light collecting optical-fiber bundle 6 is optically connected to a photomultiplier 7. The other end surface of the light collecting optical-fiber bundle 6 (which will be referred to as a "light collecting surface 60," hereinafter) so as to define a light collecting area $L_o$ of the head. The light irradiating area $L_i$ and the light collecting area $L_o$ are separated by a predetermined distance D such as several centimeters.

The light source 3 includes, for example, a plurality of lasers having light emissions of different wavelengths in the 700-1300 nanometer spectral range. The light source 3 is electrically connected to a light driver 2 which is in turn connected to a central processing unit (CPU) 1 via a bus 11.

The photomultiplier tube 7 is for increasing the intensity of the collected light and for converting the intensity into analog electric signals. The photomultiplier 7 is electrically connected to an analog-to-digital converter 8 which is in turn connected to the CPU 1 via the bus 11. A memory 9 and a display 10 are also connected to the CPU 1 via the bus 11.

The light driver 2 drives the light source 3 to generate light of different wavelengths at timing determined by the CPU 1. The light irradiating optical fiber bundle 5 transmits the light so as to irradiate the light irradiating area $L_i$ of the head 4 with the light. The light thus irradiated on the area $L_i$ penetrates into the head and the brain while being scattered and reflected by skin, soft tissue, and bone therein. A part of the light which has thus propagated in the head to reach the light collecting area $L_o$ is received by the light collecting optical-fiber bundle 6, multiplied by the photomultiplier tube 7, and converted by the analog-to-digital convertor 8 to a digital signal which represents the light intensity of the different light wavelengths reaching the light collecting area $L_o$. Calculations are performed on the resultant digital signal at the CPU 1, based on the distance D, the initial light intensities, and the like, to determine the relative levels of oxygenated hemoglobin and reduced hemoglobin which are then displayed on the display 10.

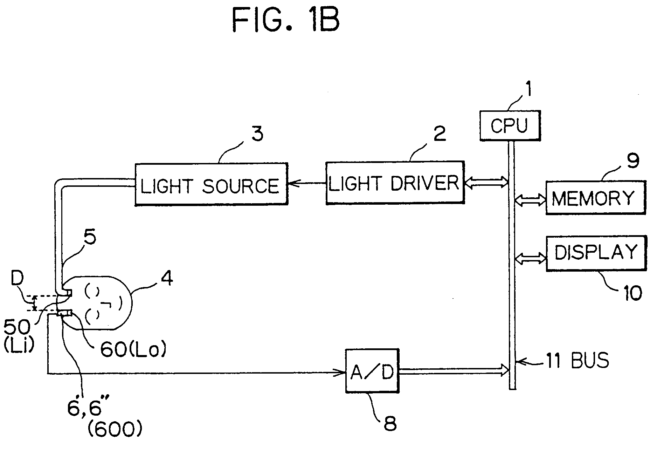

In some cases, as shown in Fig. 1B, the photomultiplier 7 (6' in this drawing) may be directly affixed to the head 4 for directly collecting the light reaching the light collecting area $L_o$. In this case, a photocathode surface of the photomultiplier 6' serves as the light collecting surface 60. In place of the photomultiplier 6', a photodiode 6" may be attached to the head 4 for collecting the light reaching the light collecting area $L_o$ and for converting the collected light intensity into the analog electrical signals. Also in this case, a photocathode surface of the photodiode 6" serves as the light collecting surface 60.

A light collecting element such as the light collecting optical-fiber bundle 6, the photomultiplier 6', the photodiode 6" for collecting light emerged from the light collecting area $L_o$ will be referred to as a "light collector 600," hereinafter. It is noted that when the optical-fiber bundle 6 is used as the light collector 600, the light collecting surface 60 is of substantially a circular shape. When the photomultiplier 6' or the photodiode 6" is used as the light collector 600, the light collecting surface 60 thereof is of substantially a square or circular shape.

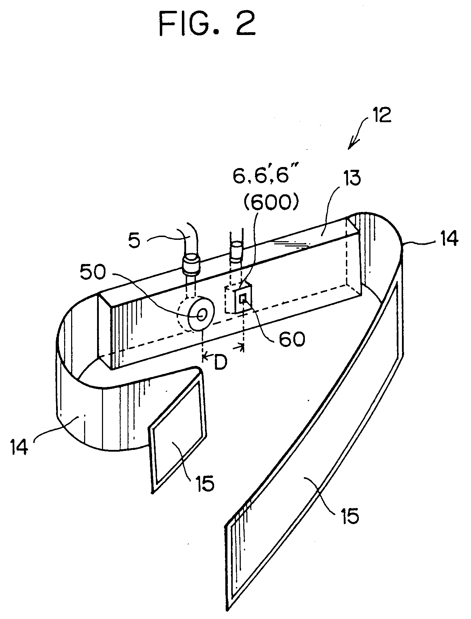

United States Patent Nos. 4,321,930, 4,380,240, and 4,510,938 describe a detachable mounting device 12 as shown in Fig. 2 for securing the light irradiating optical-fiber bundle 5 and the light collector 600 to the head 4. The mounting device 12 has a supporting portion 13 for supporting the light irradiating optical-fiber bundle 5 and the light collector 600 so that the light irradiating surface 50 and the light collecting surface 60 may be exposed. The supporting portion 13 serves to maintain the fixed distance D between the exposed light irradiating surface 50 and the exposed light collecting surface 60.

The mounting device 12 further has a pair of straps 14 and 14 for securing the supporting portion 13 to the head 4 or other body part desired to be measured. The pair of straps 14 and 14 are provided with a pair of mating "Velcro"™ type strips 15 and 15, respectively, so that the straps 14 and 14 may be easily adjusted to conform to the size and shape of the head or other body part to be measured.

Since the mounting device 12 has the above-described structure, when the mounting device 12 is mounted on the head or other body part, the light irradiating surface 50 and the light collecting surface 60 are located on the surface of the head 4 with the desired distance D being formed therebetween.

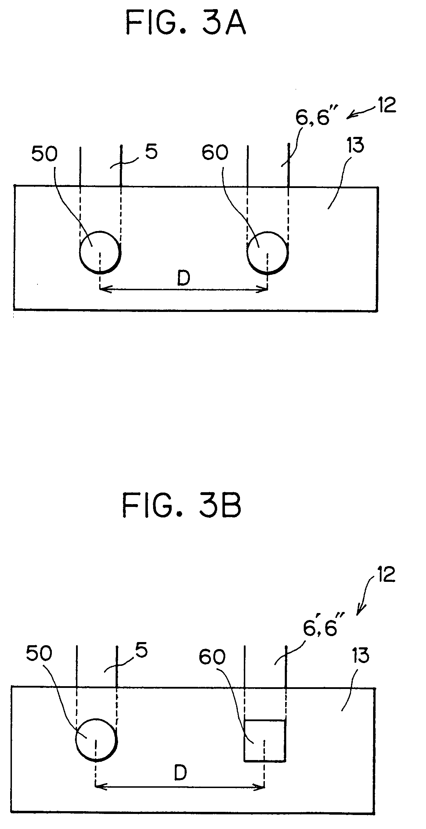

When an optical-fiber bundle 6, a photomultiplier 6', or a photodiode 6" with a substantially circular light collecting surface 60 is used, the light irradiating surface 50 and the light collecting surface 60 are arranged on the mounting device 12 as shown in Fig. 3A. When the photomultiplier 6' or the photodiode 6" with a substantially square light collecting surface 60 is used, the light irradiating surface 50 and the light collecting surface 60 are arranged as shown in Fig. 3B.

When light is irradiated from the irradiating surface 50 onto the light irradiating area $L_i$, the light penetrates into the head 4 while scattering and spreading. Deeply penetrating light picks up the most clinically important information. However, scattering and reflecting greatly weakens the light. Accordingly, when it reaches the light collecting area $L_o$, its intensity is greatly attenuated.

In order to collect as much of this deeply penetrating but attenuated light as possible, the conventional light collecting surface 60 is designed to have a large surface area. For example, a conventional photodiode 6" often have a photocathode surface 60 with a surface area of 1 cm by 1 cm.

The present inventors have found, however, that the large area of the light collecting surface 60 has the following disadvantages:

(1) The large surface area 60 raises costs of the light collector 600.
(2) The large surface area 60 defines a large light collecting area $L_o$. Accordingly, the larger the surface area 60, the vaguer the actual distance D between the irradiation point $L_i$ and the light collecting area $L_o$, which makes the measurements vaguer. The distance the light travels in the head from the irradiation point $L_i$ to the actual exit point also becomes vague, and quantifying the signal difficult.
(3) The large surface area 60 collects not only the important light but also external light which serves as an external noise disturbance.
(4) Particularly when using a photodiode 6" as the light collector 600, increasing the light collecting surface 60 increases noise from junction capacitance in the photodiode (which will be referred to as an "internal noise," hereinafter). Accordingly, detecting small amounts of light becomes difficult.
(5) In addition, the large surface 60 increases an entire size of the mounting device 12.

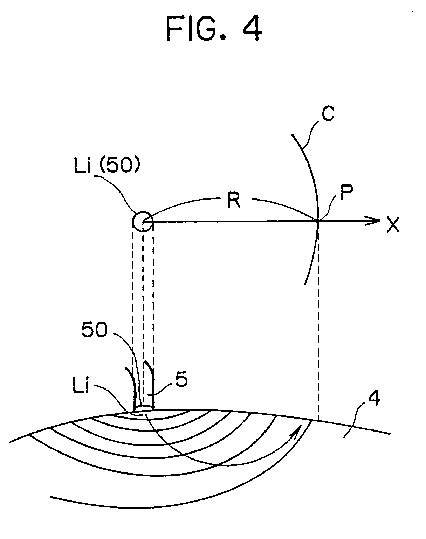

The present inventors further have noticed that intensity of the light emerging from the head is exponentially changed dependent on the distance between the light irradiating point $L_i$ and the light emerging position which may be different as it is proposed in EP-A-0 527 703 and shown particularly in Fig. 2 of this document where there is apparently a large variation in distances between points on the sensor and the light source. Now assume, on the surface of the head, a circle C with radius R on a line X is centered at the light irradiating point $L_i$ as shown in Fig. 4. Light emerges from the head at a point P on the circle C. As radius R increases, the intensity of light emerging from the point P decreases exponentially.

The above-described analysis will be applied to a concrete example relating to a square-shaped light collecting surface 60 of a photodiode located away from the light irradiating area $L_i$ in the radius direction X.

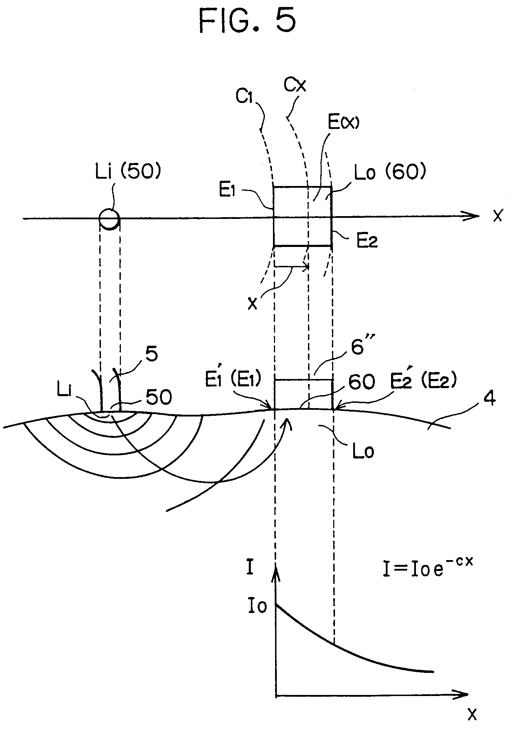

As shown in Fig. 5, the square-shaped light collecting surface 60 has a pair of opposite sides: a first side $E_1$ and a second side $E_2$, the first side $E_1$ facing the light irradiating area $L_i$. The light collecting surface 60 attached to the surface of the head defines thereon a square-shaped light collecting area $L_o$ having a first side $E'_1$ and a second side $E'_2$. The side $E'_1$ approximately lies on an arc of a circle $C_1$ centered on the light irradiating point $L_i$. Intensity of light emerged at the side $E'_1$ is assumed to have a value of $I_0$. An arbitrary line E(x) is separated from the first edge $E'_1$ by a distance x along the line X. Intensity I of light emerged at the line E(x) is therefore approximated by the following equation (1),

$$I = I_o e^{-cx} \qquad (1)$$

where c is a coefficient representative of attenuation characteristics of the light in the body to be measured. For example, if a human adult head is to be measured, c has a value of 0.31 [1/mm]. Fig. 5 also shows how the light intensity I decreases or attenuates exponentially as expressed by the equation (1). As apparent from Fig. 5, the intensity of light received by the light collecting surface 60 exponentially decreases across the light collecting surface. For example, when the light collector 600 (photodiode 6") has a 10 mm x 10 mm square light collecting surface 60, light collected at the second side $E_2$ has only one tenth of the intensity of light collected at the first side $E_1$. It is therefore apparent that only the area of the light collecting surface nearest to the light irradiating point $L_i$ collects substantial amounts of light emerged from the head, and the remaining area of the light collecting surface contributes little to the light collecting process.

According to this invention, an apparatus for use in combination with a system for non-invasively measuring an activity in a selected portion of an object, said apparatus comprises:

light irradiating means having a light irradiating portion for irradiating light onto a surface of the object;

light collecting means having a light collecting surface for collecting light which has been radiated from the light irradiating portion of said light radiating means and penetrated into and out of the selected portion in the object to emerge from the surface of the object, the light collecting surface being separated from the light irradiating portion in a radius direction of a circle centered on the light irradiating portion, this preamble being based on EP-A-0 527 703

is characterised in that the light collecting surface has a width along the radius direction and a length along a tangential direction extending perpendicularly to the radius direction, the width being shorter than the length.

The above described shape of the light collecting surface can effectively collect light deeply penetrating light emerging from the body to be measured, and therefore can enhance detecting sensitivity of the light collector.

A particular embodiment of an apparatus in accordance with the present invention will now be described in combination with the prior art with reference to the accompanying drawings, in which:-

Fig. 1A is a schematic view showing a conventional spectrophotometric reflectance apparatus;

Fig. 1B is a schematic view showing a variation of the conventional spectrophotometric reflectance apparatus shown in Fig. 1A;

Fig. 2 is a perspective view of a conventional mounting structure integrating an optical-fiber bundle and a light collector;

Fig. 3A is a schematic view showing an arrangement of the optical-fiber bundle and the light collector in the conventional mounting structure shown in Fig. 2, wherein the light collector is circular shaped;

Fig. 3B is a schematic view showing an arrangement of the optical-fiber bundle and the light collector in the conventional mounting structure shown in Fig. 2, wherein the light collector is square shaped;

Fig. 4 is a schematic view showing a positional relationship of a light irradiating surface and a light emerging surface of an object and the effects thereof on intensity of light passing from the light irradiating surface, through the object, to the light emerging surface;

Fig. 5 is a schematic view showing the effects of increasing distance from the light irradiating area on the intensity of light emerging from the light emerging area shown in Fig. 4;

Fig. 6 is a schematic diagram showing a shape and a positional relationship to the light irradiating surface of the light collector shown in Fig. 5;

Fig. 7 is a graphical representation showing theoretical increase in noise as the surface area of the light collector increases in a direction progressing away from the optical-fiber bundle;

Fig. 8 is a graphical representation showing theoretical change in signal to noise ratio across the light collector as distance form the optical-fiber bundle increases;

Fig. 9 is a graphical representation showing actual increase in noise as the surface area of the light collector increases in a direction progressing away from the optical-fiber bundle; and

Fig. 10 is graphical representation showing the actual change in signal to noise ratio across the light collector as distance form the optical-fiber bundle increases.

A preferred dimension of the width of the light collecting surface 60 relative to the length thereof can be calculated for the case where the light collector 600 is made from a photodiode.

The total light intensity collectable by the light collecting surface 60 having a rectangular shape with a width of x is expressed by the following equation (2),

$$S(x) = \int_0^x I_0 e^{-cx} dx \qquad (2)$$

If the amount of external and internal noise disturbance affecting an entire surface of the light collecting surface 60 is expressed by N(x), the signal to noise ratio (S/N) attained by the light collecting surface 60 is expressed by the following equation (3),

$$\frac{S}{N}(x) = \frac{\int_0^x I_0 e^{-cx} dx}{N(x)} \qquad (3)$$

Theoretically, the total amounts of external and internal noise disturbance are proportional to the surface area of the light collecting surface 60, and therefore proportional to the width x of the square-shaped light collecting surface 60. Accordingly, the total amount of noise disturbance obtained on the light collecting surface 60 would be expressed by the following equation (4),

$$N(x) = ax \qquad (4)$$

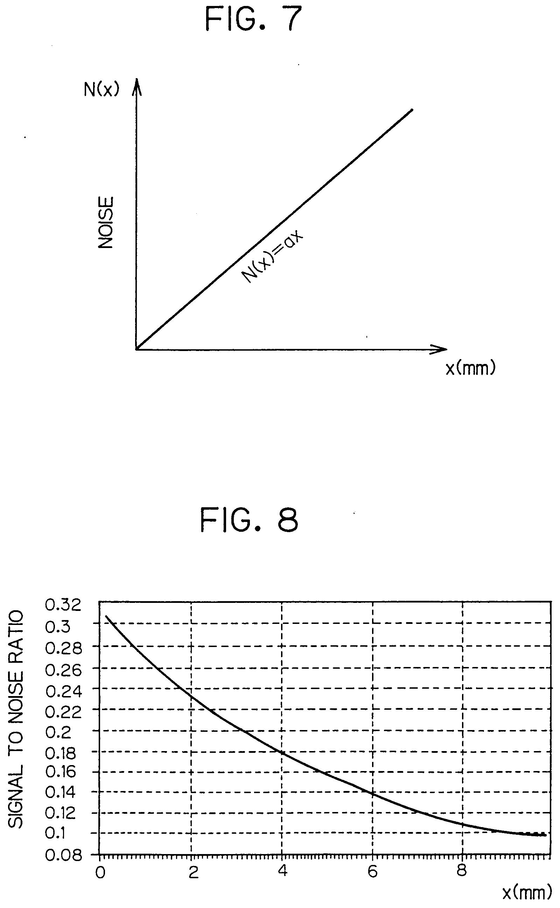

Fig. 7 shows the graph showing the relationship between the width x and the total noise N(x).

From the above-described equations (3) and (4), the theoretical signal to noise (S/N) ratio is approximated by the following equation (5),

$$\frac{S}{N}(x) = \frac{I_o}{ac}(\frac{1-e^{-cx}}{x}) \qquad (5)$$

Fig. 8 shows a graph showing the relationship between the width x and the theoretical S/N ratio (S/N (x)). As apparent from Fig. 5, the theoretical S/N ratio is greatest where x = 0. In other words, where the light collecting surface 60 has a long narrow strip shape having a width of almost zero, the light collecting surface attains the largest theoretical S/N ratio.

However, the present inventor has discovered that the internal noise disturbance does not actually decrease at the fixed rate of $\underline{a}$ as the width x decreases toward the value of zero. The total noise disturbance N(x) can therefore be approximated by the following equations (6) and (7) for example,

$$N(x)=a(\frac{1}{2}x+\frac{3}{2}) \text{ where } 0 < x < 3 \qquad (6)$$

$$N(x) = ax \text{ where } 3 < x \qquad (7)$$

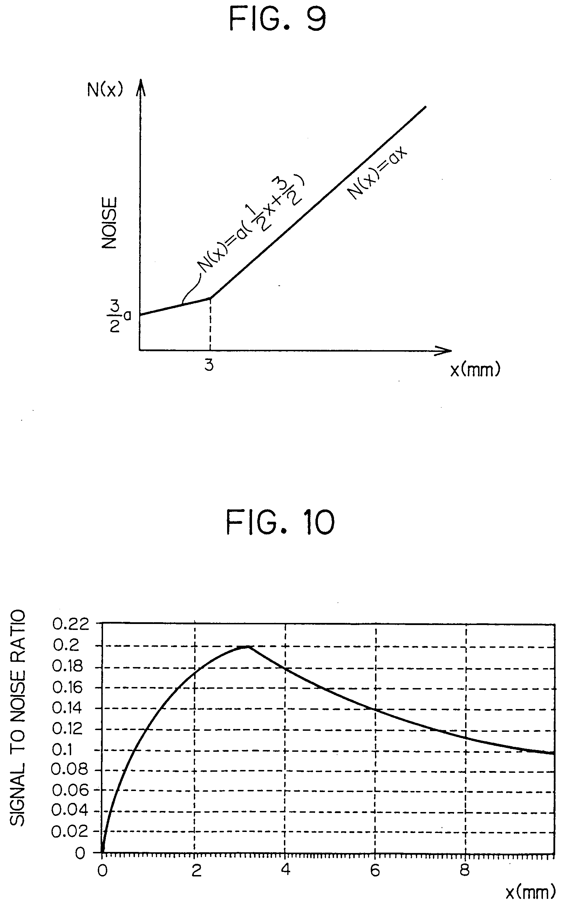

Fig. 9 is a graph showing the relationship between the actual total noise N(x) and the width x.

From the equations (3), (6) and (7), the actual signal to noise (S/N) ratio can be expressed by the following equations (8) and (9),

$$S/N(x{\geq}3) = \frac{I_o}{ac}(\frac{1-e^{-cx}}{x}) \qquad (8)$$

$$\frac{S}{N}(x{<}3) = \frac{I_o}{ac}(\frac{1-e^{-cx}}{x}) \qquad (9)$$

Fig. 10 is a graph showing the relationship between the actual S/N ratio and the width x where $I_0/ac = 1$.

Fig. 10 shows that the signal to noise ratio is greatest when the width x is about 3 mm.

Since the actual noise N(x) of the light collecting surface 60 can be approximated by the equations (6) and (7), where a photodiode of SiPIN-PD is used as the light collector 600, the S/N ratio becomes greatest where the width x is 2 to 3 mm. In other words, the optimum width x is in a range from 2 to 3 mm. Accordingly, if a length is 10 mm, the optimum surface area of the surface 60 is in a range of 20 to 30 $mm^2$.

As described above, since the width of the light collecting surface 60 is short relative to the length thereof, the following advantages are obtained:

The light collecting surface 60 can more effectively collect light emerged from the body to be measured. The external and internal noise disturbance to the light collecting surface can be decreased. Accordingly, the signal to noise ratio of the light collector can be enhanced. The decreased light collecting surface area of the light collector decreases costs of the light collector. Also, the distance between the light irradiating point and the light emerging point becomes clearer, and measurements and results more precise.

While the invention has been described in detail with reference to specific embodiments thereof, it would be apparent to those skilled in the art that various changes and modifications may be made therein without departing from the spirit of the invention.

For example, the present invention can be integrated into a conventional mountable support structure as described above for maintaining a fixed distance between the optical-fiber bundle and the light collector, with the optical-fiber bundle optically connected to a plurality of lasers having light emissions of different wavelengths in the 700-1300 nanometer spectral range.

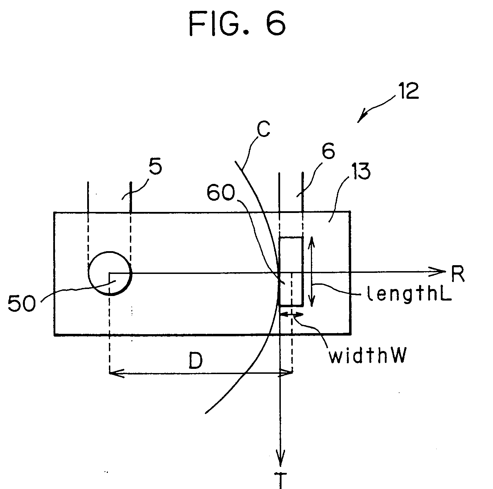

Although the present invention has been described in the embodiment as a photodiode, it is equally applicable to a optical-fiber bundle, a photomultiplier including dynodes, or any other type of light collector. Accordingly, although Fig. 6 shows the example of the present invention where the light collecting surface 60 has the rectangular shape with its width W being shorter than its length L, the light collecting surface 60 may have an oval shape with its width W being shorter than its length L.

Furthermore, although the light collecting surface 60 shown in Fig. 6 has a rectangular shape, the first side $E_1$ could have an arcuate shape substantially conforming to the curve of the circle C. The second side $E_2$ could also have an arcuate shape substantially parallel to the curve of the circle C.

Of course, the present invention can be used in a conventional system as shown in Figs. 1A and 1B for non-invasively monitoring various functions in the body, such as blood flow, blood volume, or metabolic rate in the brain.

## Claims

1. An apparatus for use in combination with a system for non-invasively measuring an activity in a selected portion of an object (4), said apparatus comprising:

   light irradiating means (50) having a light irradiating portion for irradiating light onto a surface of the object (4);
   light collecting means (600) having a light collecting surface (60) for collecting light which has been radiated from the light irradiating portion of said light radiating means (50) and penetrated into and out of the selected portion in the object (4) to emerge from the surface of the object (4), the light collecting surface (60) being separated from the light irradiating portion in a radius direction (X) of a circle centered on the light irradiating portion,

   characterised in that the light collecting surface (60) has a width (W) along the radius direction (X) and a length (L) along a tangential direction extending perpendicularly to the radius direction (X), the width (W) being shorter than the length (L).

2. The apparatus as claimed in claim 1, further comprising a support structure (12) detachably mountable to the object (4) for supporting said light irradiating means (50) and said light collecting means (600) so as to maintain a fixed distance between the light irradiating portion (50) and the light collecting surface (60) along the radius direction.

3. An apparatus as claimed in claim 1 or 2, wherein the length (L) of the light collecting surface (60) is substantially 10 mm and the width (W) is in a range from substantially 2 mm to 3 mm.

4. An apparatus as claimed in any preceding claim, wherein the light collecting surface has an area having a value in a range of 20 mm$^2$ to 30 mm$^2$.

5. An apparatus as claimed in any preceding claim, wherein said light collecting means (600) further includes light intensity increasing means such as a photo-multiplier (7) for increasing intensity of the light collected by said light collecting surface (60), and wherein said light collecting surface (60) comprises the photocathode surface of the photo-multiplier (7), or a light collecting surface of an optical fiber bundle (6).

6. An apparatus as claimed in any one of claims 1 to 4, wherein said light collecting means (600) includes a photodiode (6"), the light collecting surface (60) comprising the photocathode surface of the photodiode (6").

7. An apparatus as claimed in any one of the preceding claims, wherein said light irradiating means includes a light source (3) and an optical fiber bundle (5) optically connected to the light source (3), the optical fiber bundle (5) having the light irradiating portion.

8. An apparatus as claimed in claim 7, wherein the light source includes a plurality of lasers having light emissions of different wavelengths in the 700 - 1300 nanometer spectral range.

9. A system for non-invasively measuring an activity in a selected portion of an object comprising an apparatus in accordance with any one of the preceding claims, and calculation means (1) for calculating the activity in the selected portion of the object, based on the light collected by said light collecting means (600).

10. An apparatus or system as claimed in any one of the preceding claims, wherein said light irradiating means (50) and said light collecting means (600) are adapted for monitoring an in vivo organ such as the brain as the selected portion.

**Patentansprüche**

1. Vorrichtung zum Gebrauch in Kombination mit einem System zum nicht-invasiven Messen einer Aktivität in einem ausgewählten Teil eines Objektes (4), wobei diese Vorrichtung aufweist:

   lichtausstrahlende Mittel (50), die einen lichtausstrahlenden Teil zum Strahlen von Licht auf eine Oberfläche des Objektes (4) haben; lichtsammelnde Mittel (600), die eine lichtsammelnde Oberfläche (60) zum Sammeln von Licht haben, welches von dem lichtausstrahlenden Teil des lichtausstrahlenden Mittels (50) ausgestrahlt worden und in den ausgewählten Teil des Objekts (4) eingedrungen und aus diesem herausgekommen ist, um aus der Oberfläche des Objektes (4) auszutreten, wobei die lichtsammelnde Oberfläche (60) von dem lichtausstrahlenden Teil in Radialrichtung (X) eines Kreises getrennt ist, der auf den lichtausstrahlenden Teil zentriert ist,

   dadurch gekennzeichnet, daß die lichtsammelnde Oberfläche (60) eine Breite (W) entlang der Radialrichtung (X) und eine Länge (L) entlang der sich senkrecht zu der Radialrichtung (X) erstreckenden Tangentialrichtung hat, wobei die Breite (W) kürzer als die Länge (L) ist.

2. Vorrichtung gemäß Anspruch 1, die zudem eine Trägeranordnung (12) aufweist, die lösbar an dem Objekt (4) befestigbar ist, um das lichtausstrahlende Mittel (50) zu tragen und das lichtsammelnde Mittel (600), um einen festen Abstand zwischen dem lichtausstrahlenden Teil (50) und der lichtsammelnden Oberfläche (60) in Radialrichtung aufrechtzuerhalten.

3. Eine Vorrichtung wie in Anspruch 1 oder 2 beansprucht, bei der die Länge (L) der lichtsammelnden Oberfläche (60) im wesentlichen 10 mm beträgt und die Breite (W) in einem Bereich von im wesentlichen 2 mm bis 3 mm ist.

4. Vorrichtung wie in irgendeinem der vorstehenden Ansprüche beansprucht, bei der die lichtsammelnde Oberfläche eine Fläche hat, die eine Größe in einem Bereich von 20 mm$^2$ bis 30 mm$^2$ hat.

5. Vorrichtung wie in irgendeinem der vorstehenden Ansprüche beansprucht, bei der die lichtsammelnden Mittel (600) außerdem die Lichtintensität steigernde Mittel so wie einen Photovervielfacher (7) zum Verstärken der Intensität des durch die lichtsammelnde Oberfläche (60) gesammelten Lichtes einschließen, und in der die lichtsammelnde Oberfläche (60) die Oberfläche der Photokathode des Photovervielfachers (7) einschließt, oder eine lichtsammelnde Oberfläche eines optischen Faserbündels (6).

6. Vorrichtung wie in irgendeinem der Ansprüche 1 bis 4 beansprucht, bei der die lichtsammelnden Mittel (600) eine Photodiode (6") einschließen, wobei die lichtsammelnde Oberfläche (60) die Photokathodenoberfläche der Photodiode (6") umfaßt.

7. Vorrichtung wie in irgendeinem der vorstehenden Ansprüche beansprucht, bei der die lichtausstrahlenden Mittel eine Lichtquelle (3) einschließen und ein optisches Faserbündel (5), das optisch mit der Lichtquelle (3) verbunden ist, wobei das optische Faserbündel (5) den lichtausstrahlenden Teil hat.

8. Vorrichtung wie in Anspruch 7 beansprucht, bei der die Lichtquelle eine Vielzahl von Lasern einschließt, die Lichtemissionen von verschiedenen Wellenlängen in dem Spektralbereich von 700 bis 1300 Nanometer haben.

9. Ein System zum nicht-invasiven Messen einer Aktivität in einem ausgewählten Teil eines Objektes, das eine Vorrichtung in Übereinstimmung mit irgendeinem der vorstehenden Ansprüche aufweist, und Rechenmittel (1) zum Berechnen Aktivität in dem ausgewählten Teil des Objektes, basierend auf dem Licht, das durch die lichtsammelnden Mittel (600) gesammelt ist.

10. Eine Vorrichtung oder ein System wie in irgendeinem der vorstehenden Ansprüche beansprucht, bei dem die lichtausstrahlenden Mittel (50) und die lichtsammelnden Mittel (600) angepaßt sind, um ein lebendes Organ zu überwachen, so wie das Gehirn als das ausgewählte Teil.

**Revendications**

1. Appareil destiné à être utilisé en combinaison avec un système pour mesurer, de façon non invasive, une activité dans une partie sélectionnée d'un objet (4), ledit appareil comprenant :

des moyens d'irradiation de lumière (50) ayant une partie d'irradiation de lumière pour irradier de la lumière sur une surface de l'objet (4) ; des moyens de collecte de lumière (600) ayant une surface de collecte de lumière (60) pour collecter la lumière qui a été irradiée par la partie d'irradiation de lumière desdits moyens d'irradiation de lumière (50) et qui a pénétré dans et en dehors de la partie sélectionnée de l'objet (4) pour émerger de la surface de l'objet (4), la surface de collecte de lumière (60) étant séparée de la partie d'irradiation de lumière dans une direction radiale (X) d'un cercle centré sur la partie d'irradiation de lumière,

caractérisé en ce que la surface de collecte de lumière (60) a une largeur (W) le long de la direction radiale (X) et une longueur (L) le long d'une direction tangentielle s'étendant perpendiculairement à la direction radiale (X), la largeur (W) étant plus courte que la longueur (L).

2. Appareil selon la revendication 1, comprenant en outre une structure de support (12) pouvant être montée de façon amovible sur l'objet (4) pour supporter lesdits moyens d'irradiation de lumière (50) et lesdits moyens de collecte de lumière (600) de manière à maintenir une distance fixe entre la partie d'irradiation de lumière (50) et la surface de collecte de lumière (60) le long de la direction radiale.

3. Appareil selon la revendication 1 ou 2, dans lequel la longueur (L) de la surface de collecte de lumière (60) est sensiblement de 10 mm et la largeur (W) se trouve dans une plage s'étendant sensiblement de 2 mm à 3 mm.

4. Appareil selon l'une quelconque des revendications précédentes, dans lequel la surface de collecte de lumière a une aire ayant une valeur qui se trouve dans la plage s'étendant de 20 mm$^2$ à 30 mm$^2$.

5. Appareil selon l'une quelconque des revendications précédentes, dans lequel lesdits moyens de collecte de lumière (600) comprennent en outre des moyens d'accroissement d'intensité de la lumière tels qu'un photomultiplicateur (7) pour accroître l'intensité de la lumière collectée par ladite surface de collecte de lumière (60), et dans lequel ladite surface de collecte de lumière (60) comprend la surface de photocathode du photomultiplicateur (7), ou une surface de collecte de lumière d'un faisceau de fibres optiques (6).

6. Appareil selon l'une quelconque des revendications 1 à 4, dans lequel lesdits moyens de collecte de lumière (600) comprennent une photodiode (6"), la surface de collecte de lumière (60) comprenant la surface de photocathode de la photodiode (6").

7. Appareil selon l'une quelconque des revendications précédentes, dans lequel lesdits moyens d'irradiation de lumière comprennent une source de lumière (3) et un faisceau de fibres optiques (5) optiquement connectées à la source de lumière (3), le faisceau de fibres optiques (5) comportant la partie d'irradiation de lumière.

8. Appareil selon la revendication 7, dans lequel la source de lumière comprend une pluralité de lasers ayant des émissions de lumière de différentes longueurs d'onde, dans la plage spectrale s'étendant de 700 à 1300 nanomètres.

9. Système pour mesurer, de façon non invasive, une activité dans une partie sélectionnée d'un objet, comprenant un appareil selon l'une quelconque des revendications précédentes et des moyens de calculs (1) pour calculer l'activité dans la partie sélectionnée de l'objet, sur la base de la lumière collectée par lesdits moyens de collecte de lumière (600).

10. Appareil ou système selon l'une quelconque des revendications précédentes, dans lesquels lesdits moyens d'irradiation de lumière (50) et lesdits moyens de collecte de lumière (600) sont adaptés pour contrôler un organe in vivo tel qu'un cerveau en tant que partie sélectionnée.

# FIG. 1A

# FIG. 1B

# FIG. 2

# FIG. 3A

# FIG. 3B

# FIG. 4

# FIG. 5

$$I = I_0 e^{-cx}$$

# FIG. 6

# FIG. 7

# FIG. 8

# FIG. 9

# FIG. 10